# EUROPEAN PATENT APPLICATION

(11) **EP 4 147 630 A1**
(43) Date of publication of application: **15.03.2023**
(21) Application number: 22194611.4
(22) Date of filing: 08.09.2022
(51) Int. Cl.: A61B 5/00, A61B 5/01, A61B 5/0205, A61B 5/024, A61B 5/11, A61B 7/00

(54) **MATERNAL AND FETAL MONITORING DEVICE USING MULTIPLE SENSING UNITS**

(30) Priority: 11.09.2021 US 202163243109 P; 05.09.2022 US 202217902964
(71) Applicant: SiriuXense Co., Ltd., East Dist. Hsinchu City 300 (TW)
(72) Inventor: Tsai, Fu-Ji, 220 New Taipei City (TW); Huang, Ji-De, 300 Hsinchu City (TW); Hung, Ju-Yu, 413 Taichung City (TW); Kuo, Chen-I, 114 Taipei City (TW); Lu, Chih-Chien, 334 Taoyuan City (TW)
(74) Representative: Becker Kurig & Partner Patentanwälte mbB

(57) **Abstract**

A maternal and fetal monitoring device using multiple sensing units is disclosed. The maternal and fetal monitoring device comprises: a processor module and a plurality of sensor modules, wherein each said sensor module comprises: an inertial sensor, a temperature sensor and a first acoustic sensor. After being attached onto a maternal body, each said sensor module collects a body temperature signal, an inertia signal and a sound signal. Subsequently, the processor module determines a maternal body posture by analyzing the inertia signal, determines a maternal physical condition and a fetal physical condition by analyzing the inertial signal, the plurality of first sound signals and the second sound signal, and estimates physiological parameters of the maternal body and a fetus by analyzing the body temperature sensing signal, the plurality of first sound signals, and the second sound signals.

## Description

### CROSS-REFERENCE TO RELATED APPLICATIONS

This application claims benefits of U. S. Provisional Patent Application Ser. No. 63/243,109 for "Hybrid and location-based, real-time, portable, wearable, and fully integrated fetal monitoring system", filed September 11, 2021. The contents of which are hereby incorporated by reference in its entirety for all purposes.

### BACKGROUND OF THE INVENTION

### 1. Field of the Invention

The present invention relates to the technology field of an electronic device configured for monitoring physical conditions and/or physiological parameters from a maternal body, and more particularly to a maternal and fetal monitoring device using multiple sensing units.

### 2. Description of the Prior Art

There have been a variety of maternal/fetal monitoring systems proposed. For example, U.S. patent No. 10,039,459B2 discloses a pregnant human subject monitoring system, U.S. patent No. 6,551,251B1 discloses a fetal heart monitoring system, and U.S. patent publication No. 2018/0256057A1 discloses a fetal phonocardiogram system.

According to the disclosures of U.S. patent No. 10,039,459B2, the pregnant human subject monitoring system is configured for monitoring the wellbeing of a fetus by the non-invasive detection and analysis of fetal cardiac electrical activity data. However, the disclosed pregnant human subject monitoring system fails to simultaneously measure physiological parameters (e.g., maternal respiratory rate) of the maternal body and determine physical conditions (e.g., fetal movement) of the fetus and the maternal body. On the other hand, according to the disclosures of U.S. patent No. 6,551,251B1, the fetal heart monitoring system is configured for detecting and processing acoustic fetal heart signals from a maternal body, so as to eventually estimate fetal heart activities. However, the disclosed fetal heart monitoring system is not allowed for achievement in measuring physiological parameters (e.g., maternal respiratory rate) of the maternal body and determining physical conditions (e.g., fetal movement) of the fetus and the maternal body. Furthermore, according to the disclosures of U.S. patent publication No. 2018/0256057A1, the fetal phonocardiogram system is configured for utilizing a piezoelectric transducer to collect a sound signal from a maternal body, processing the sound signal to a fetal heart sound sensing signal, and eventually processing the fetal heart sound sensing signal so as to estimate a fetal heart rate of a fetus. It is a pity that, the disclosed fetal phonocardiogram system also fails to measure physiological parameters (e.g., maternal respiratory rate) of the maternal body and determine physical conditions (e.g., fetal movement) of the fetus and the maternal body.

According to above descriptions, it is understood that there are still rooms for improvement in the conventional maternal/fetal monitoring system. In view of this fact, inventors of the present application have made great efforts to make inventive research and eventually provided a maternal and fetal monitoring device using multiple sensing units.

### SUMMARY OF THE INVENTION

The primary objective of the present invention is to disclose a maternal and fetal monitoring device using multiple sensing units, which is adopted for being attached onto a maternal body, thereby not only determining a maternal physical condition and a fetal physical condition, and but also simultaneously measuring physiological parameters of the maternal body and a fetus.

For achieving the primary objective mentioned above, the present invention provides an embodiment of the maternal and fetal monitoring device, which is adopted for being attached onto a maternal body, and comprises:
a processor module, comprising a first body provided with a circuit assembly therein, wherein the circuit assembly comprises a microprocessor, a memory and a wireless transmission interface; and
a plurality of sensor modules, being coupled to the processor module, wherein each said sensor module comprises a second body provided with first acoustic sensor assembly therein, and the first acoustic sensor assembly comprising an inertial sensor, a temperature sensor and a first acoustic sensor;
wherein the memory stores an application program including instructions, such that in case the application program is executed, the microprocessor being configured for:
   controlling said temperature sensor to measure a body temperature from the maternal body, thereby generating a body temperature sensing signal;
   controlling said inertial sensor to monitor a movement and/or a vibration of the maternal body, thereby generating an inertial signal;
   controlling the plurality of first acoustic sensor to collect a sound emitted from the maternal body, thereby correspondingly generating a plurality of first sound signals;
   analyzing the body temperature sensing signal, the inertial signal and the plurality of first sound signals, so as to determine a maternal physical condition, a maternal body posture, and a fetal physical condition as well as estimate at least one physiological parameter of the maternal body and a fetus; and
   transmitting a real-time data containing the maternal physical condition, the maternal body posture, the fetal physical condition, and the at least one physiological parameter to an electronic device by using the wireless transmission interface.

In one embodiment, the maternal body posture is selected from a group consisting of lying on a back of the maternal body, lying on a left side of the maternal body, lying on a right side of the maternal body, and sitting upright.

In one embodiment, the maternal physical condition is selected from a group consisting of maternal organ sound, maternal speaking sound, vibration sound of umbilical artery, and vibration sound of uterine artery, and the physiological parameter of the maternal body is selected from a group consisting of maternal heart rate, maternal body temperature, and maternal respiration.

In one embodiment, the fetal physical condition is selected from a group consisting of fetal movements, fetal hiccups and fetal position, and the physiological parameter of the fetus is selected from a group consisting of fetal heart sounds and fetal heartbeat.

In one embodiment, the electronic device is selected from a group consisting of signal transceiver device, tablet computer, cloud server, laptop computer, desktop computer, all-in-one computer, smart phone, smart watch, and smart glasses.

In one embodiment, the memory is selected from a group consisting of embedded flash (eFlash) memory, flash memory chip, hard drive (HD), solid state drive (SSD), and USB flash drive.

In a practicable embodiment, a device fixing member is allowed to be used for making the maternal and fetal monitoring device be attached onto the maternal body, so as to make each said second body contact the maternal body by a body contacting surface thereof.

In one embodiment, the first body has a first accommodation space for receiving the circuit assembly, and each said second body has a second accommodation space for receiving the first acoustic sensor assembly.

In one embodiment, an aperture is formed on a bottom of the second accommodation space, such that the first acoustic sensor of the first acoustic sensor assembly is exposed out of the second body via the aperture.

In one embodiment, a circular recess is formed on the body contacting surface of the second body, and the circular recess has a depth and a diameter in a range between 4.5 mm and 20 mm, such that a ratio of the diameter to the depth is not greater than 6.

In one embodiment, the second body has a Shore hardness in a range between A20 and A50, and a manufacture material of the second body is selected from a group consisting of liquid silicone rubber (LSR), silicon rubber, silicone, acrylonitrile butadiene styrene (ABS), polyurethane (PU), and polydimethylsiloxane (PDMS).

In one embodiment, a body connecting member is connected between the first body and each said second body, and the body connecting member is provided with an electrical connecting component therein, such that the circuit assembly is coupled to the first acoustic sensor assembly through the electrical connecting component.

In one practicable embodiment, the maternal and fetal monitoring device further comprises:
a wireless charging module, being coupled to the circuit assembly; and
a battery, being coupled to the circuit assembly;
wherein the battery is allowed to be electrically charged by the wireless charging module or a battery charger.

In another one practicable embodiment, the maternal and fetal monitoring device further comprises:
a second acoustic sensor assembly coupled to the circuit assembly, being disposed in the first body and/or said second body, and comprising a second acoustic sensor;
wherein the second acoustic sensor is adopted for collecting an ambient sound, thereby generating and transmitting a second sound signal to the processor module;
wherein the sound collected by the first acoustic sensor has a sound frequency in a range between 0.1 Hz and 500 Hz, and the ambient sound collected by the second acoustic sensor has a sound frequency in a range between 16 Hz and 2K Hz.

In one embodiment, the first acoustic sensor assembly is coupled to the circuit assembly via a first flexible printed circuit board, and the second acoustic sensor assembly is coupled to the circuit assembly through a second flexible printed circuit board.

In one embodiment, the application program consists of a plurality of subprograms, and the plurality of subprograms comprising:
a first subprogram, being compiled to be integrated in the application program by one type of programming language, and including instructions for configuring the microprocessor to control the temperature sensor to collect the body temperature sensing signal from the maternal body;
a second subprogram, being compiled to be integrated in the application program by one type of programming language, and including instructions for configuring the microprocessor to control each said first acoustic sensor to collect the sound emitted from the maternal body, and to control the second acoustic sensor to collect the ambient sound;
a third subprogram, being compiled to be integrated in the application program by one type of programming language, and including instructions for configuring the microprocessor to control the inertial sensor to monitor the movement and/or the vibration of the maternal body;
a fourth subprogram, being compiled to be integrated in the application program by one type of programming language, and including instructions for configuring the microprocessor to apply a signal synchronizing process to the body temperature sensing signal, the inertial signal, the plurality of first sound signals, and the second sound signal according to timestamps that are respectively contained in the body temperature sensing signal, the inertial signal, the first sound signal, and the second sound signal;
a fifth subprogram, being compiled to be integrated in the application program by one type of programming language, and including instructions for configuring the microprocessor to apply a signal process to the body temperature sensing signal, the inertial signal, the plurality of first sound signals, and/or the second sound signal;
a sixth subprogram, being compiled to be integrated in the application program by one type of programming language, and including instructions for configuring the microprocessor to analyze the inertial signal, the plurality of first sound signals, and the second sound signal, so as to determine the maternal physical condition and the fetal physical condition;
a seventh subprogram, being compiled to be integrated in the application program by one type of programming language, and including instructions for configuring the microprocessor to analyze the inertial signal, so as to determine the maternal body posture;
an eighth subprogram, being compiled to be integrated in the application program by one type of programming language, and including instructions for configuring the microprocessor to analyze the body temperature sensing signal, the plurality of first sound signals, and the second sound signal, so as to estimate the at least one physiological parameter of the maternal body and the fetus; and
a ninth subprogram, being compiled to be integrated in the application program by one type of programming language, and including instructions for configuring the microprocessor to utilize the wireless transmission interface 11F to transmit a warning signal to the electronic device in case of at least one of the maternal physical condition, the maternal body posture, the fetal physical condition, and the at least one physiological parameter showing abnormal.

In one practicable embodiment, the plurality of subprograms further comprises:
a tenth subprogram, being compiled to be integrated in the application program by one type of programming language, and including instructions for configuring the microprocessor to judge whether there is a well contact between the second body and the maternal body by analyzing a vibration of said maternal respiration, said maternal body temperature, and a first frequency band and a second frequency band of the first sound signal.

### BRIEF DESCRIPTION OF THE DRAWINGS

The invention as well as a preferred mode of use and advantages thereof will be best understood by referring to the following detailed description of an illustrative embodiment in conjunction with the accompanying drawings, wherein:
FIG. 1 shows a block diagram of a maternal and fetal monitoring device using multiple sensing units according to the present invention;
FIG. 2A shows a first stereo diagram of the maternal and fetal monitoring device according to the present invention;
FIG. 2B shows a second stereo diagram of the maternal and fetal monitoring device according to the present invention;
FIG. 3A shows a third stereo diagram of the maternal and fetal monitoring device according to the present invention;
FIG. 3B shows a fourth stereo diagram of the maternal and fetal monitoring device according to the present invention;
FIG. 4A shows a first exploded view of the maternal and fetal monitoring device according to the present invention;
FIG. 4B shows a second exploded view of the maternal and fetal monitoring device according to the present invention;
FIG. 5 shows a block diagram of a microprocessor, a memory, a wireless transmission interface, a temperature sensor, an inertial sensor, a first acoustic sensor, and a second acoustic sensor they are shown in FIG. 4A and FIG. 4B;
FIG. 6 shows a fifth stereo diagram of the maternal and fetal monitoring device according to the present invention;
FIG. 7 shows a measured data graph of an inertial signal, a body temperature sensing signal and a first sound signal;
FIG. 8 shows a measured data graph of three first sound signals correspondingly transmitted from three sensor modules; and
FIG. 9 shows a measured data graph of an inertial signal transmitted from an inertial sensor and three first sound signals correspondingly transmitted from three sensor modules.

### DETAILED DESCRIPTION OF THE PREFERRED EMBODIMENTS

To more clearly describe a maternal and fetal monitoring device using multiple sensing units according to the present invention, embodiments of the present invention will be described in detail with reference to the attached drawings hereinafter.

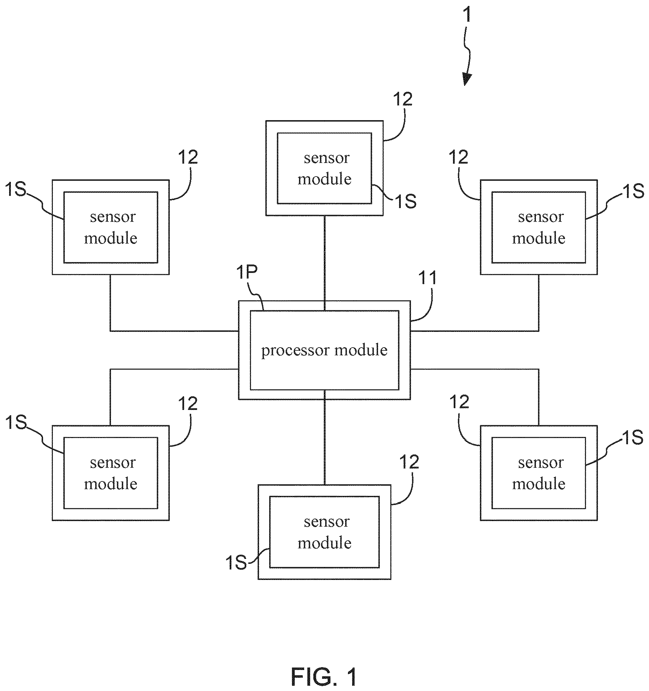

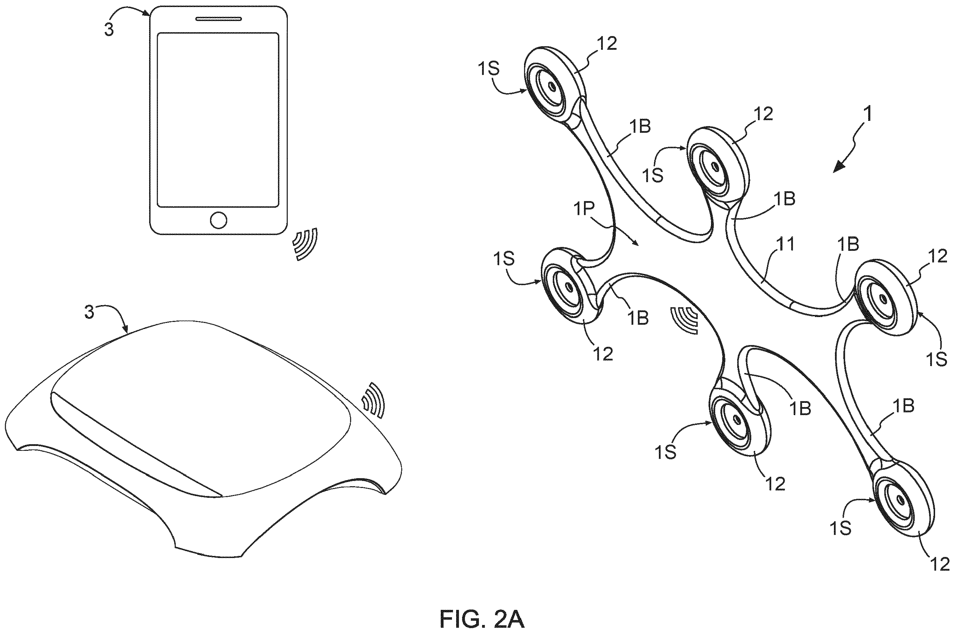

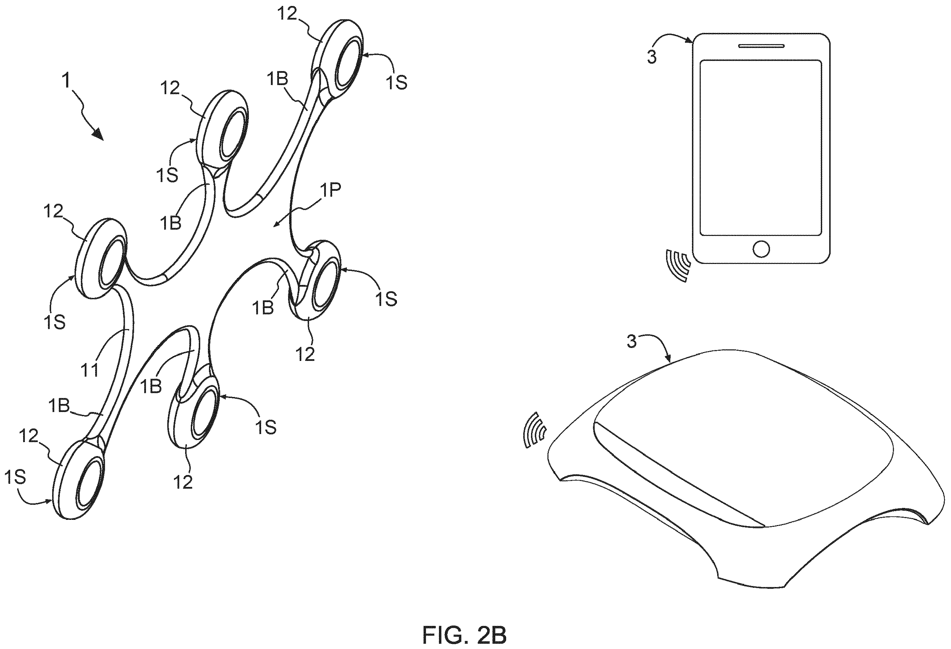

With reference to FIG. 1, there is provided a block diagram of a maternal and fetal monitoring device using multiple sensing units according to the present invention. Moreover, FIG. 2A and FIG. 2B shows stereo diagrams of the maternal and fetal monitoring device. As FIG. 1, FIG. 2A and FIG. 2B shows, the present invention discloses a maternal and fetal monitoring device 1 using multiple sensing units ("maternal and fetal monitoring device 1", hereinafter), which is adopted for being attached onto a maternal body, so as to not only determine a maternal physical condition and a fetal physical condition and but also simultaneously measure physiological parameters of the maternal body and a fetus. According to the present invention, the maternal and fetal monitoring device 1 comprises a processor module 1P and a plurality of sensor modules 1S. After being attached onto a maternal body, each said sensor module 1S collects a body temperature sensing signal, an inertia signal, and a first sound signal from the maternal body, and also collects a second sound signal from the ambient. Subsequently, the processor module 1P determines a maternal body posture by analyzing the inertia signal, estimates a maternal body temperature by analyzing the body temperature sensing signal, determines a maternal physical condition and a fetal physical condition by analyzing the inertial signal, the plurality of first sound signals and the second sound signal, and estimates physiological parameters of the maternal body and a fetus by analyzing the plurality of first sound signals, and the second sound signals.

In one embodiment, the maternal body posture determinable for the maternal and fetal monitoring device 1 includes: lying on a left side of the maternal body, lying on a right side of the maternal body, and sitting upright. On the other hand, the maternal physical condition determinable for the maternal and fetal monitoring device 1 includes: maternal organ sound, maternal speaking sound, vibration sound of umbilical artery, and vibration sound of uterine artery. Moreover, the physiological parameters of the maternal body measurable for the maternal and fetal monitoring device 1 include: maternal heart rate, maternal body temperature, and maternal respiration. In addition, the fetal physical condition determinable for the maternal and fetal monitoring device 1 includes: fetal movements, fetal hiccups and fetal position. Moreover, the physiological parameter of the fetus measurable for the maternal and fetal monitoring device 1 includes: fetal heart sounds and fetal heartbeat.

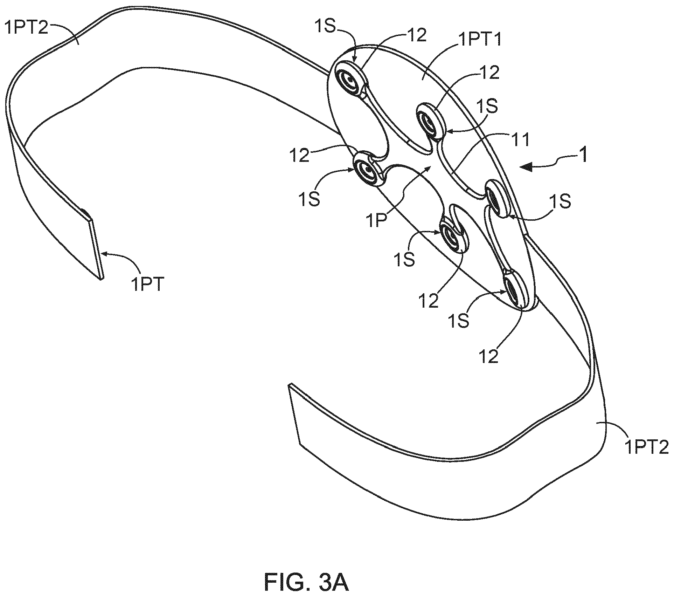

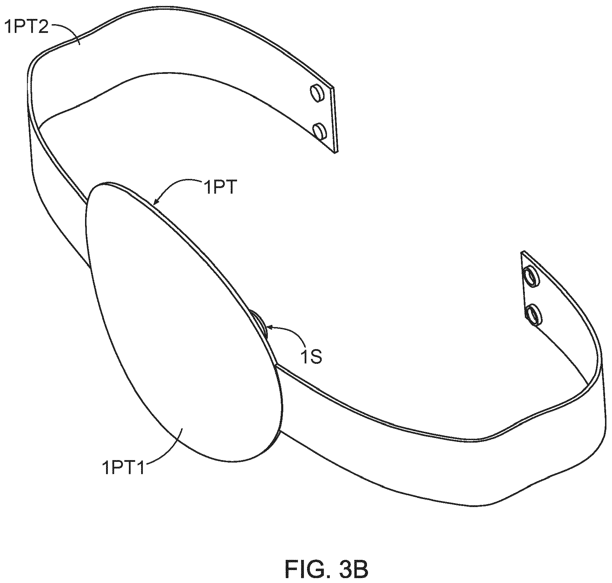

FIG. 3A shows a third stereo diagram of the maternal and fetal monitoring device, and FIG. 3B illustrates a fourth stereo diagram of the maternal and fetal monitoring device. When using this maternal and fetal monitoring device 1, a device fixing member 1PT is allowed to be used for making the maternal and fetal monitoring device 1 be attached onto the maternal body, so as to make each said second body 12 contact the maternal body by a body contacting surface thereof. Specifically, FIG. 3A depicts that the device fixing member 1PT comprises a device supporting part 1PT1 and two straps 1PT2. When using this maternal and fetal monitoring device 1, user is able to firstly disposed the maternal and fetal monitoring device 1 on the device supporting part 1PT1 of the device fixing member 1PT, and wraps the two straps 1PT2 around the waist of the maternal body, so as to make the device supporting part 1PT1 face to the abdomen of the maternal body. As such, each said sensor module 1S of the maternal and fetal monitoring device 1 is positioned to contact the abdominal skin of the maternal body by the device fixing member 1PT.

According to the present invention, in case of the fact that maternal physical condition, the maternal body posture, the fetal physical condition, and the at least one physiological parameter show abnormal, the microprocessor 11P generates a warning signal, and then transmits the warning signal to an electronic device 3 like a signal transceiver device through a wireless transmission interface 11F. Besides the signal transceiver device, the electronic device 3 can be a cloud server, a local server belong to a hospital, a postpartum center or an infant care center, and can also be a personal electronic device belong to the baby's parent, wherein the personal electronic device can be a tablet computer, a laptop computer, a desktop computer, an all-in-one computer, a smart phone, a smart watch, or a smart glasses.

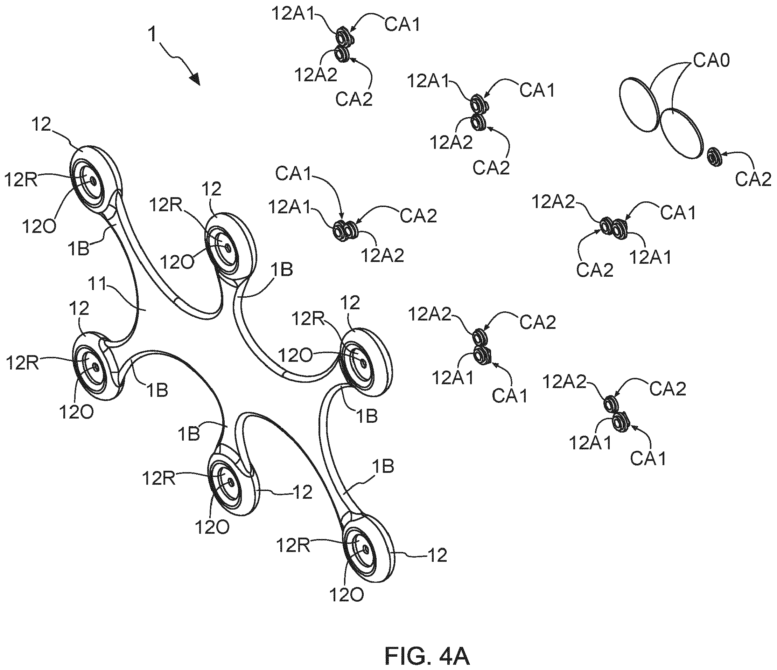

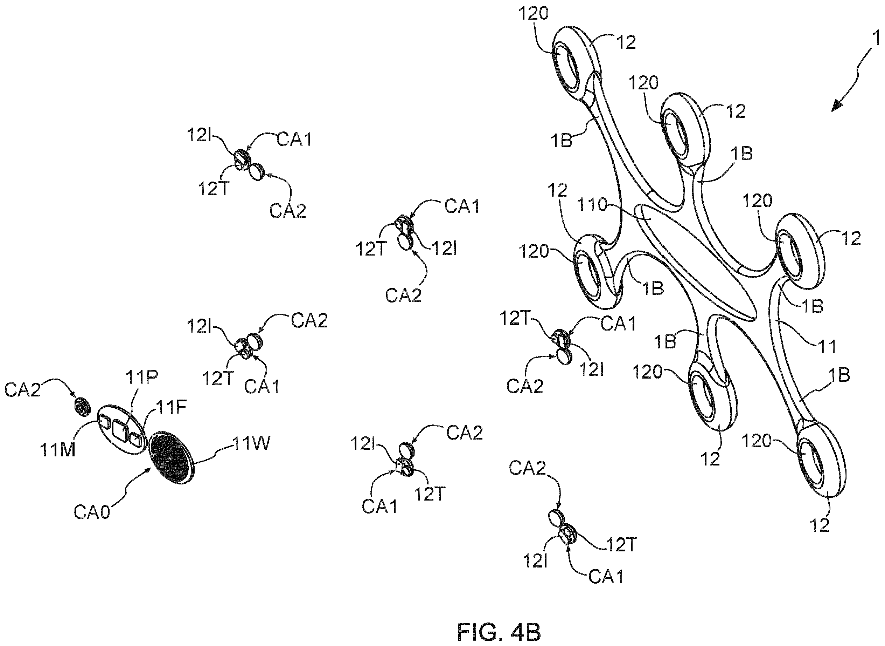

FIG. 4A shows a first exploded view of the maternal and fetal monitoring device, and FIG. 4B illustrates a second exploded view of the maternal and fetal monitoring device. As FIG. 2A, FIG. 2B, FIG. 4A, and FIG. 4B show, the processor module 1P comprises a first body 11 provided with a circuit assembly CA0 therein, wherein the circuit assembly CA0 comprises a microprocessor 11P, a memory 11M and a wireless transmission interface 11F. Moreover, the plurality of sensor modules 1S are coupled to the processor module 1P. According to the present invention, each said sensor module 1S comprises a second body 12 provided with a first acoustic sensor assembly CA1 therein, wherein the first acoustic sensor assembly CA1 comprises an inertial sensor 121, a temperature sensor 12T and a first acoustic sensor 12A1. In a practicable embodiment, a second acoustic sensor assembly CA2 is arranged to be disposed in the first body 11 and/or said second body 12. As FIG. 4A and FIG. 4B show, the second acoustic sensor assembly CA2 is coupled to the circuit assembly CA0, and includes a second acoustic sensor 12A2. According to the present invention, the second acoustic sensor 12A2 is adopted for collecting an ambient sound, thereby generating and transmitting a second sound signal to the processor module 1P.

As described in more detail below, the first acoustic sensor 12A1 is adopted for collecting fetal hiccups sound, fetal heart sound, fetal heartbeat sound, maternal organ sound, maternal speaking sound, metal heartbeat sound, maternal respiration sound, vibration sound of umbilical artery, and vibration sound of uterine artery. Since the foregoing multiple types of sounds all have a sound frequency below 500 Hz, the first acoustic sensor 12A1 is configured for collecting the sound that is emitted from the maternal body and has a sound frequency in a range between 0.1 Hz and 500 Hz. In addition, the first acoustic sensor 12A1 is also adopted for collecting sound of primitive reflexes actions and sound of irregular fetal movement from the fetus via the maternal body. Furthermore, in order to make sure that the sound collected by the first acoustic sensor 12A1 indeed contains target sound features like the feature of fetal heart sound, the second acoustic sensor 12A2 is utilized to collect an ambient sound having a sound frequency in a range between 16 Hz and 2K Hz.

As FIG. 2A, FIG. 2B, FIG. 4A, and FIG. 4B show, there is body connecting member 1B connected between the first body 11 and each said second body 12, wherein the body connecting member 1B is provided with an electrical connecting component therein, such that the circuit assembly CA0 is coupled to the first acoustic sensor assembly CA1 accommodated in the second body 12 through the electrical connecting component. In addition, the maternal and fetal monitoring device 1 of the present invention further comprises a wireless charging module 11W and a battery (not shown) both coupled to the circuit assembly CA0. By such arrangements, the battery is allowed to be electrically charged by the wireless charging module 11W or a battery charger. In a practicable embodiment, a first flexible printed circuit board and a second flexible printed circuit board can be integrated in the maternal and fetal monitoring device 1, thereby making the first acoustic sensor assembly CA1 be coupled to the circuit assembly CA0 via the first flexible printed circuit board, and making the second acoustic sensor assembly CA2 be coupled to the circuit assembly CA0 through the second flexible printed circuit board. As such, the first sound signal (containing low-frequency maternal heart sound, fetal heart sound and multiple types of vibration sounds) and the second sound signal (containing ambient sound) are decoupled during being transmitted to the microprocessor 11P of the circuit assembly CA0. Therefore, by comparing the first sound signal with the second sound signal, it is able to verify whether the first sound signal contains target sound features like the feature of fetal heart sound or not.

As described in more detail below, the first body 11 has a first accommodation space 110 for receiving the circuit assembly CA0, and each said second body 12 has a second accommodation space 120 for receiving the first acoustic sensor assembly CA1, wherein an aperture 120 is formed on a bottom of the second accommodation space 120, such that the first acoustic sensor 12A1 of the first acoustic sensor assembly CA1 is exposed out of the second body 12 via the aperture 120. According to the present invention, a circular recess 12R is formed on the body contacting surface of the second body 12, and the circular recess 12R has a depth and a diameter in a range between 4.5 mm and 20 mm, such that a ratio of the diameter to the depth being not greater than 6. In a practicable embodiment, a manufacture material of the second body 12 can be liquid silicone rubber (LSR), silicon rubber, silicone, acrylonitrile butadiene styrene (ABS), polyurethane (PU), or polydimethylsiloxane (PDMS), and the second body 12 is designed to have a Shore hardness in a range between A20 and A50.

It is worth explaining that, after the second body 12 is set to contact the maternal body by a body contacting surface thereof, the circular recess 12R helps the body contacting surface to well contact the skin of the abdominal skin of the maternal body with high air tightness, thereby making an acoustic coupling path be formed between the second body 12 and a sound source portion of the maternal body (e.g., abdominal cavity). It is worth further explaining that the human body is a low frequency resonator. Therefore, in case of there being a sound (e.g., fetal heart sound, sound of fetal movements) produced in the maternal body, magnitude of the low frequency band of the sound would be amplified by the low frequency resonator. Moreover, because there is an acoustic coupling path formed between the second body 12 and the abdominal cavity (i.e., sound source portion) of the maternal body, the sound emitted by the maternal body is directly collected by the first acoustic sensor 12A1 through the acoustic coupling path.

In a specific embodiment, the depth can be designed to have a minimum value of 1.5 mm. On the other hand, in case of the second body 12 being set to contact the maternal body by the body contacting surface thereof, the circular recess 12R is also allowed to prevent the aperture 120 (i.e., sound collecting hole for the first acoustic sensor 12A1) from being plugged by the abdominal skin of the maternal body. Specifically, the second body 12 is made of LSR so as to have a Shore hardness in a range between A20 and A50. Therefore, in spite of the fact that the second body 12 has contacted the abdominal skin of the maternal body for a period of long time, the abdominal skin of the maternal body still not shows the symptoms of allergy, itching and/or redness. On the other hand, since the circular recess 12R is a softness and elasticity cavity without diaphragm, it not only assists the first acoustic sensor 12A1 in effectively collect the sound (e.g., fetal heart sound, sound of fetal movements) emitted from the maternal body, but also amplified the magnitude of the low frequency band of the sound. Moreover, since the circular recess 12R is a low-frequency resonant cavity without diaphragm, the contact area between the second body 2 and the abdominal skin of the maternal body is significantly reduced. As such, in spite of the fact that the second body 12 has contacted the abdominal skin of the maternal body for a period of long time, the abdominal skin would not become a sultry or skin.

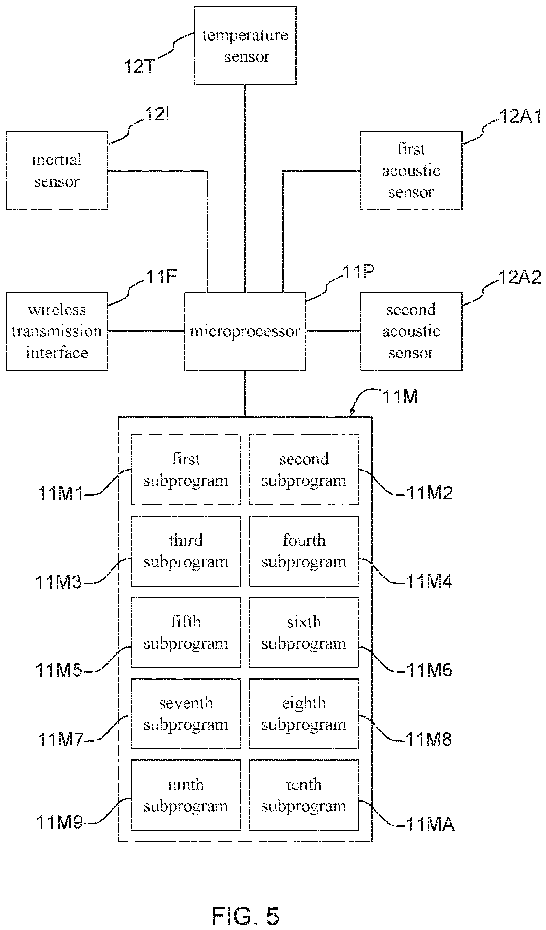

FIG. 5 shows a block diagram of the microprocessor 11P, the memory 11M, the wireless transmission interface 11F, the temperature sensor 12T, the inertial sensor 121, the first acoustic sensor 12A1, and the second acoustic sensor 12A2 they are shown in FIG. 4A and FIG. 4B. In one embodiment, the memory 12M stores an application program including instructions, such that in case the application program is executed, the microprocessor 11P is configured for controlling the temperature sensor 12T, the inertial sensor 121, the first acoustic sensor 12A1, the second acoustic sensor 12A2, and the wireless transmission interface 11F, so as to achieve the determination of a maternal physical condition and a fetal physical condition and the measurement of physiological parameters of the maternal body and the fetus. As FIG. 5 shows, the application program consists of a plurality of subprograms, and the plurality of subprograms comprising: a first subprogram 11M1, a second subprogram 11M2, a third subprogram 11M3, a fourth subprogram 11M4, a fifth subprogram 11M5, a sixth subprogram 11M6, a seventh subprogram 11M7, an eighth subprogram 11M8, a ninth subprogram 11M9, and a tenth subprogram 11MA.

It is worth explaining that, not only does the memory 11M can be an embedded flash (eFlash) memory provided in the microprocessor 11P, but the memory 11M can also be a flash memory chip, a hard drive (HD), a solid state drive (SSD), or an USB flash drive that is coupled to the microprocessor 11P.

According to the present invention, the first subprogram 11M1 is compiled to be integrated in the application program by one type of programming language, and includes instructions for configuring the microprocessor 11P to control the temperature sensor 12T to measure a body temperature from the maternal body, thereby generating a body temperature sensing signal. On the other hand, the second subprogram 11M2 is compiled to be integrated in the application program by one type of programming language, and includes instructions for configuring the microprocessor 11P to control each said first acoustic sensor 12A1 to collect the sound emitted from the maternal body, and to control the second acoustic sensor 12A2 to collect the ambient sound. Moreover, the third subprogram 11M3 is compiled to be integrated in the application program by one type of programming language, and includes instructions for configuring the microprocessor 11P to control the inertial sensor to monitor a movement and/or a vibration of the maternal body, thereby generating an inertial signal. In addition, the fourth subprogram 11M4 is compiled to be integrated in the application program by one type of programming language, and includes instructions for configuring the microprocessor 11P to apply a signal synchronizing process to the body temperature sensing signal, the inertial signal, the plurality of first sound signals, and the second sound signal according to timestamps that are respectively contained in the body temperature sensing signal, the inertial signal, the first sound signal, and the second sound signal. Moreover, the fifth subprogram 11M5 is compiled to be integrated in the application program by one type of programming language, and includes instructions for configuring the microprocessor 11P to apply a signal process to the body temperature sensing signal, the inertial signal, the plurality of first sound signals, and/or the second sound signal.

As described in more detail below, the sixth subprogram 11M6 is compiled to be integrated in the application program by one type of programming language, and includes instructions for configuring the microprocessor 11P to analyze the inertial signal, the plurality of first sound signals, and the second sound signal, so as to determine a maternal physical condition and a fetal physical condition. On the other hand, the seventh subprogram 11M7 is compiled to be integrated in the application program by one type of programming language, and includes instructions for configuring the microprocessor 11P to analyze the inertial signal, so as to determine a maternal body posture. Moreover, the eighth subprogram 11M8 is compiled to be integrated in the application program by one type of programming language, and includes instructions for configuring the microprocessor 11P to analyze the body temperature sensing signal, the plurality of first sound signals, and the second sound signal, so as to estimate the at least one physiological parameter of the maternal body and the fetus. In addition, the ninth subprogram 11M9 is compiled to be integrated in the application program by one type of programming language, and includes instructions for configuring the microprocessor 11P to utilize the wireless transmission interface 11F to transmit a warning signal to the electronic device 3 in case of at least one of the maternal physical condition, the maternal body posture, the fetal physical condition, and the at least one physiological parameter showing abnormal.

Particularly, there is further a tenth subprogram 11MA stored in the memory 11M. The tenth subprogram 11MA is compiled to be integrated in the application program by one type of programming language, and includes instructions for configuring the microprocessor 11P to judge whether there is a well contact between the second body 12 and the maternal body by analyzing a vibration of said maternal respiration, said maternal body temperature, and a first frequency band and a second frequency band of the first sound signal. According to the particular design of the present invention, before starting to monitor the physical conditions and/or measure the physiological parameters from the maternal body, the tenth subprogram 11MA is executed by the microprocessor 11P, such that the microprocessor 11P is configured to judge whether there is a well contact between the second body 12 and the maternal body or not. After the second body 12 is detected, by the sensor modules IS, to have already had a well contact with the abdominal skin of the maternal body, the microprocessor 11P immediately enables the maternal and fetal monitoring device 1 to work normally. By such design, the foregoing well-contact detecting function is not only helpful in making the maternal and fetal monitoring device 1 to achieve the monitoring of a maternal physical condition and a fetal physical condition and the measurement of physiological parameters of the maternal body and the fetus, but also significantly save the power consumption of the maternal and fetal monitoring device 1.

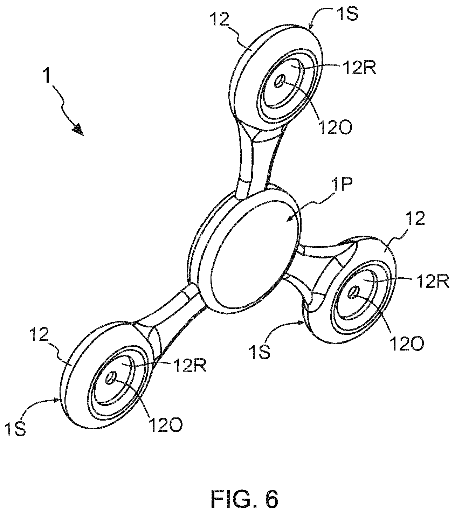

With reference to FIG. 6, there is shown a fifth stereo diagram of the maternal and fetal monitoring device. In an exemplary embodiment, the maternal and fetal monitoring device 1 of the present invention consists of three sensor modules 1S and one processor module 1P. When using this maternal and fetal monitoring device 1, the three sensor modules 1S are firstly set to contact a maternal body by a body contacting surface thereof. Subsequently, after each said the second body 12 is detected to have already had a well contact with the maternal body, the microprocessor 11P immediately enables to work normally, so as to monitor a maternal physical condition and a fetal physical condition, and simultaneously measure physiological parameters of the maternal body and the fetus.

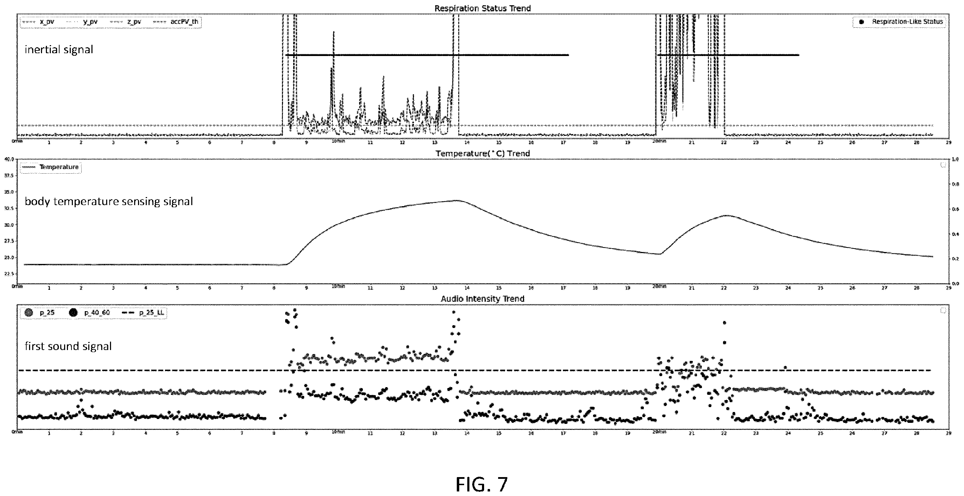

FIG. 7 shows a measured data graph of an inertial signal, a body temperature sensing signal and a first sound signal. In case the tenth subprogram 11MA is executed, the microprocessor 11P is configured to firstly determine whether the inertial signal includes a signal segment for describing a breath variation of the maternal body. In other words, existing of said signal segment in the inertial signal means that the second body 12 of the sensor module 1S have already had a well contact with the maternal bodyy. On the other hand, it is also practicable to judge whether there is a well contact between the second body 12 and the maternal body by analyzing the body temperature sensing signal. For example, in case the second body 12 is set to well contact the maternal body by a body contacting surface, there is an obviously signal variation occurring in the body temperature sensing signal (as shown in FIG. 7). Of course, if there is a signal variation suddenly occurring in the body temperature sensing signal in case of the maternal and fetal monitoring device 1 being operated, it means that the second body 12 is no longer having a well contact with the maternal body. Particularly, it is also practicable to judge whether there is a well contact between the second body 12 and the maternal body analyzing the first sound signal. According to the measured data graph of FIG. 7, when the second body 12 is set to well contact the maternal body, the magnitude of a first frequency band in the first sound signal shows an abruptly enhancement, wherein the first frequency band of the first sound signal includes the sound emitted from the maternal body falls below the 25 Hz frequency band. Moreover, According to the measured data graph of FIG. 7, when the second body 12 is set to well contact the maternal body, the magnitude of a second frequency band in the first sound signal also shows an abruptly enhancement, wherein the second frequency band of the first sound signal includes the sound emitted from the baby's body 2 falls between 40 Hz and 60 Hz.

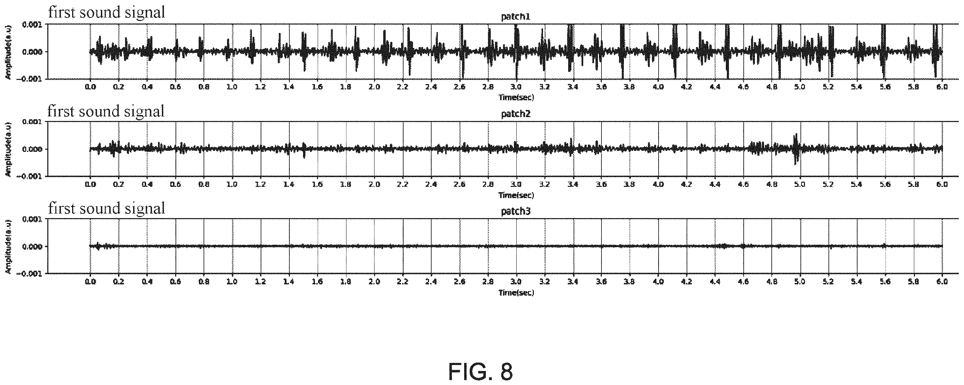

FIG. 8 shows a measured data graph of three first sound signals correspondingly transmitted from three sensor modules. It is worth explaining that, the data graph of FIG. 8 are obtained by using the maternal and fetal monitoring device 1 as shown in FIG. 6 to collect the sound emitted from a maternal body at 37 weeks pregnant. Engineers skilled in development and manufacture of fetal heart sound monitoring device certainly know that the fetal heart sound has a sound frequency below 150 Hz. For example, there is literature indicating that the sound frequency of the fetal heart sound is 2.5 Hz. Therefore, the first acoustic sensor 12A1 is configured for collecting the sound that is emitted from the maternal body and has a sound frequency in a range between 0.1 Hz and 500 Hz, such that the first sound signal transmitted by the first acoustic sensor 12A1 would contain the target sound features like the feature of fetal heart sound. According to the measured data graph of FIG. 8, it is able to see that there is an obvious feature of fetal heart sound existing in the first sound signal that is transmitted by the sensor module patch 1. On the other hand, there is only weak feature of fetal heart sound existing in the first sound signal that is transmitted by the sensor module patch 2. However, there is no feature of fetal heart sound existing in the first sound signal that is transmitted by the sensor module patch 3.

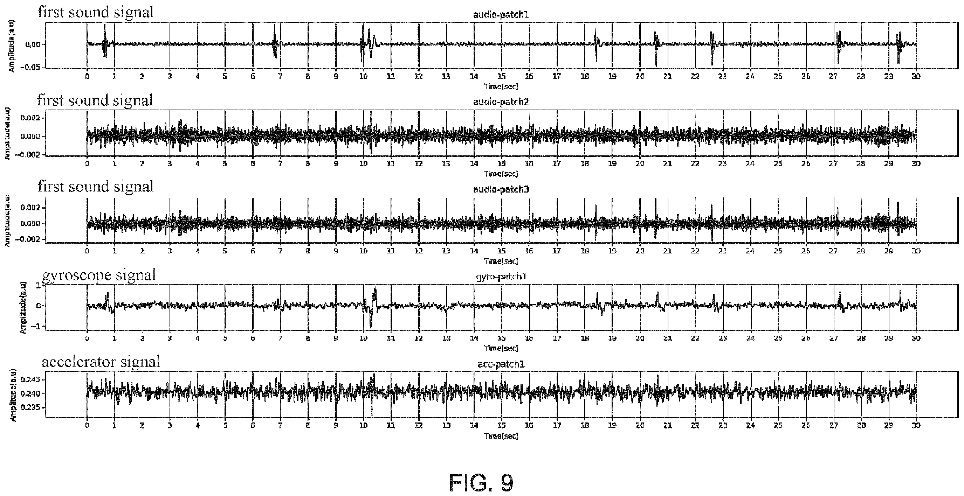

FIG. 9 shows a measured data graph of an inertial signal transmitted from an inertial sensor and three first sound signals correspondingly transmitted from three sensor modules. It is worth explaining that, the data graph of FIG. 9 are obtained by using the maternal and fetal monitoring device 1 as shown in FIG. 6 to collect the sound emitted from a maternal body at 38 weeks pregnant. Research reports have indicated that, primitive reflexes actions and irregular fetal movements made by a fetus would produce low-frequency sound. Therefore, the first acoustic sensor 12A1 is configured for collecting the sound that is emitted from the maternal body and has a sound frequency in a range between 0.1 Hz and 500 Hz, such that the first sound signal transmitted by the first acoustic sensor 12A1 would contain the feature of fetal movements sound. Moreover, the inertial sensor 121 can also sense corresponding vibrations of the primitive reflexes actions and irregular fetal movements, thereby generating a gyroscope signal and an accelerator signal. According to the measured data graph of FIG. 9, significant feature of fetal movements sound can be extracted, by peak detection, from the first sound signal that is transmitted by the sensor module patch 1. On the other hand, there is only weak feature of fetal movements sound allowed to be extracted out from the first sound signal that is transmitted by the sensor module patch 3. However, there is no feature of fetal movements sound existing in the first sound signal that is transmitted by the sensor module patch 2. It is worth noting that, the gyroscope signal and the accelerator signal both contain the feature of fetal movements sound.

It needs to further explain that, the microprocessor 11P is provided with an analog-to-digital (A/D) convertor therein. After the microprocessor 11P receives the body temperature sensing signal, the inertial signal, the plurality of first sound signals, and the second sound signal, the A/D convertor is enabled to apply an analog-to-digital conversion process to the foregoing signals. As described in more detail below, the A/D convertor directly digitize the first sound signal, digitize the second sound signal using a first sampling rate, and digitize the inertial signal using a second sampling rate. In one embodiment, the first sampling rate is not greater than 4 KHz (i.e., ≦4 KHz), and the second sampling rate is not greater than 120 Hz (i.e., ≦ 120 Hz). After that, the microprocessor 11P executes the fifth subprogram 11M5, so as to process the first sound signal, the second sound signal, and/or the inertial signal. For example, the microprocessor 11P applies a FFT (fast Fourier transform) process to the foregoing signals.

Therefore, through above descriptions, all embodiments and their constituting elements of the maternal and fetal monitoring device using multiple sensing units according to the present invention have been introduced completely and clearly. Moreover, the above description is made on embodiments of the present invention. However, the embodiments are not intended to limit the scope of the present invention, and all equivalent implementations or alterations within the spirit of the present invention still fall within the scope of the present invention.

## Claims

1. A maternal and fetal monitoring device adopted for being attached onto a maternal body, comprising:
a processor module, comprising a first body provided with a circuit assembly therein, wherein the circuit assembly comprises a microprocessor, a memory and a wireless transmission interface; and
a plurality of sensor modules, being coupled to the processor module, wherein each said sensor module comprises a second body provided with a first acoustic sensor assembly therein, and the first acoustic sensor assembly comprising an inertial sensor, a temperature sensor and a first acoustic sensor;
wherein the memory stores an application program including instructions, such that in case the application program is executed, the microprocessor being configured for:
controlling said temperature sensor to measure a body temperature from the maternal body, thereby generating a body temperature sensing signal;
controlling said inertial sensor to monitor a movement and/or a vibration of the maternal body, thereby generating an inertial signal;
controlling the plurality of first acoustic sensor to collect a sound emitted from the maternal body, thereby correspondingly generating a plurality of first sound signals;
analyzing the body temperature sensing signal, the inertial signal and the plurality of first sound signals, so as to determine a maternal physical condition, a maternal body posture, and a fetal physical condition as well as estimate at least one physiological parameter of the maternal body and a fetus; and
transmitting a real-time data containing the maternal physical condition, the maternal body posture, the fetal physical condition, and the at least one physiological parameter to an electronic device by using the wireless transmission interface.

2. The maternal and fetal monitoring device of claim 1, wherein the maternal body posture is selected from a group consisting of lying on a back of the maternal body, lying on a left side of the maternal body, lying on a right side of the maternal body, and sitting upright.

3. The maternal and fetal monitoring device of claim 1, wherein the maternal physical condition is selected from a group consisting of maternal organ sound, maternal speaking sound, vibration sound of umbilical artery, and vibration sound of uterine artery, and the physiological parameter of the maternal body being selected from a group consisting of maternal heart rate, maternal body temperature, and maternal respiration.

4. The maternal and fetal monitoring device of claim 1, wherein the fetal physical condition is selected from a group consisting of fetal movements, fetal hiccups and fetal position, and the physiological parameter of the fetus being selected from a group consisting of fetal heart sounds and fetal heartbeat.

5. The maternal and fetal monitoring device of claim 1, wherein the electronic device is selected from a group consisting of signal transceiver device, tablet computer, cloud server, laptop computer, desktop computer, all-in-one computer, smart phone, smart watch, and smart glasses.

6. The maternal and fetal monitoring device of claim 1, wherein the memory is selected from a group consisting of embedded flash (eFlash) memory, flash memory chip, hard drive (HD), solid state drive (SSD), and USB flash drive.

7. The maternal and fetal monitoring device of claim 3, wherein a device fixing member is allowed to be used for making the maternal and fetal monitoring device be attached onto the maternal body, so as to make each said second body contact the maternal body by a body contacting surface thereof.

8. The maternal and fetal monitoring device of claim 7, wherein the first body has a first accommodation space for receiving the circuit assembly, and each said second body having a second accommodation space for receiving the first acoustic sensor assembly.

9. The maternal and fetal monitoring device of claim 8, wherein an aperture is formed on a bottom of the second accommodation space, such that the first acoustic sensor of the first acoustic sensor assembly is exposed out of the second body via the aperture.

10. The maternal and fetal monitoring device of claim 9, wherein a circular recess is formed on the body contacting surface of the second body, and the circular recess having a depth and a diameter in a range between 4.5 mm and 20 mm, such that a ratio of the diameter to the depth is not greater than 6.

11. The maternal and fetal monitoring device of claim 10, wherein the second body has a Shore hardness in a range between A20 and A50, and a manufacture material of the second body being selected from a group consisting of liquid silicone rubber (LSR), silicon rubber, silicone, acrylonitrile butadiene styrene (ABS), polyurethane (PU), and polydimethylsiloxane (PDMS).

12. The maternal and fetal monitoring device of claim 9, wherein a body connecting member is connected between the first body and each said second body, and the body connecting member being provided with an electrical connecting component therein, such that the circuit assembly is coupled to the first acoustic sensor assembly through the electrical connecting component.

13. The maternal and fetal monitoring device of claim 9, further comprising:
a wireless charging module, being coupled to the circuit assembly; and
a battery, being coupled to the circuit assembly;
wherein the battery is allowed to be electrically charged by the wireless charging module or a battery charger.

14. The maternal and fetal monitoring device of claim 9, further comprising:
a second acoustic sensor assembly coupled to the circuit assembly, being disposed in the first body and/or said second body, and comprising a second acoustic sensor;
wherein the second acoustic sensor is adopted for collecting an ambient sound, thereby generating and transmitting a second sound signal to the processor module;
wherein the sound collected by the first acoustic sensor has a sound frequency in a range between 0.1 Hz and 500 Hz, and the ambient sound collected by the second acoustic sensor having a sound frequency in a range between 16 Hz and 2K Hz.

15. The maternal and fetal monitoring device of claim 14, wherein the first acoustic sensor assembly is coupled to the circuit assembly via a first flexible printed circuit board, and the second acoustic sensor assembly being coupled to the circuit assembly through a second flexible printed circuit board.

16. The maternal and fetal monitoring device of claim 14, wherein the application program consists of a plurality of subprograms, and the plurality of subprograms comprising:
a first subprogram, being compiled to be integrated in the application program by one type of programming language, and including instructions for configuring the microprocessor to control the temperature sensor to collect the body temperature sensing signal from the maternal body;
a second subprogram, being compiled to be integrated in the application program by one type of programming language, and including instructions for configuring the microprocessor to control each said first acoustic sensor to collect the sound emitted from the maternal body, and to control the second acoustic sensor to collect the ambient sound;
a third subprogram, being compiled to be integrated in the application program by one type of programming language, and including instructions for configuring the microprocessor to control the inertial sensor to monitor the movement and/or the vibration of the maternal body;
a fourth subprogram, being compiled to be integrated in the application program by one type of programming language, and including instructions for configuring the microprocessor to apply a signal synchronizing process to the body temperature sensing signal, the inertial signal, the plurality of first sound signals, and the second sound signal according to timestamps that are respectively contained in the body temperature sensing signal, the inertial signal, the first sound signal, and the second sound signal;
a fifth subprogram, being compiled to be integrated in the application program by one type of programming language, and including instructions for configuring the microprocessor to apply a signal process to the body temperature sensing signal, the inertial signal, the plurality of first sound signals, and/or the second sound signal;
a sixth subprogram, being compiled to be integrated in the application program by one type of programming language, and including instructions for configuring the microprocessor to analyze the inertial signal, the plurality of first sound signals, and the second sound signal, so as to determine the maternal physical condition and the fetal physical condition;
a seventh subprogram, being compiled to be integrated in the application program by one type of programming language, and including instructions for configuring the microprocessor to analyze the inertial signal, so as to determine the maternal body posture;
an eighth subprogram, being compiled to be integrated in the application program by one type of programming language, and including instructions for configuring the microprocessor to analyze the body temperature sensing signal, the plurality of first sound signals, and the second sound signal, so as to estimate the at least one physiological parameter of the maternal body and the fetus; and
a ninth subprogram, being compiled to be integrated in the application program by one type of programming language, and including instructions for configuring the microprocessor to utilize the wireless transmission interface to transmit a warning signal to the electronic device in case of at least one of the maternal physical condition, the maternal body posture, the fetal physical condition, and the at least one physiological parameter showing abnormal.

17. The maternal and fetal monitoring device of claim 16, wherein the plurality of subprograms further comprises:
a tenth subprogram, being compiled to be integrated in the application program by one type of programming language, and including instructions for configuring the microprocessor to judge whether there is a well contact between the second body and the maternal body by analyzing a vibration of said maternal respiration, said maternal body temperature, and a first frequency band and a second frequency band of the first sound signal.
